# EUROPEAN PATENT APPLICATION

(11) **EP 4 755 391 A1**
(43) Date of publication of application: **10.06.2026**
(21) Application number: 25220901.0
(22) Date of filing: 04.12.2025
(51) Int. Cl.: A61K 38/00, A61K 38/08, A61K 38/17, A61P 25/16

(54) **THE PATH TO A CURE FOR PARKINSON'S DISEASE**

(30) Priority: 05.12.2024 HU 2400558
(71) Applicant: HUN-REN Természettudományi Kutatóközpont, Veszprém (HU)
(72) Inventor: Ovádi, Judit Magdolna, Budapest (HU); Oláh, Judit, Budapest (HU); Lehotzky, Attila Ákos, Budapest (HU)
(74) Representative: Danubia Patent & Law Office LLC

(57) **Abstract**

Neurological disorders, Parkinson's disease and Multiple System Atrophy (MSA) cause serious socio-economic problems as there are, at present only therapies that treat the symptoms. Parkinson's research is a highly competitive field focusing on the specific drug targeting of the aggregation of alpha-synuclein (SYN). The discovery of Tubulin Polymerization Promoting Protein (TPPP) was a crucial factor in anti-Parkinson research. TPPP is a prominent pathological partner of SYN, which shifts the SYN-SYN aggregation toward the formation of fatal SYN-TPPP assemblies. The disassembly of the SYN-TPPP by specific agents may eliminate the toxicity of the pathological assembly of SYN by the proteolytic degradation of the excess SYN and leads to the recovery of the physiologically active proteins.

## Description

### FIELD OF THE INVENTION

The invention relates to agents for use in inhibiting the formation of or disintegrating the alpha-synuclein - alpha-synuclein (SYN-SYN) complex, wherein the agent inhibits or disrupts the association of tubulin polymerization promoting protein (TPPP) and SYN. In particular, the invention relates to agents directly inhibiting or disrupting the association of SYN and TPPP for use in inhibiting the formation of or disintegrating the alpha-synuclein - alpha-synuclein (SYN-SYN) complex.

### BACKGROUND OF THE INVENTION

Parkinson's disease (PD) is characterized by motor dysfunction, neuronal loss mainly in dopaminergic neurons in the substantia nigra pars compacta and the accumulation of alpha-synuclein (SYN) aggregates. Neurological disorders such as PD and Multiple System Atrophy (MSA) cause serious socio-economic problems as there are, at present, only therapies that treat the symptoms. Parkinson's research is a highly competitive field focusing on the specific drug targeting of the aggregation (seed) of SYN. The role of the pathological aggregation of SYN in the development of PD has been revealed, and the inhibition of SYN-SYN associations (self-assembly) has been influenced by specific small molecules, specific antibody, mRNA/siRNA and PROTAC technologies at both in vitro and in vivo (mice, C. elegans) conditions. Although these treatments could be effective concerning the disassembly of the SYN assemblies; however, the effect of the SYN disassembly was not reflected at clinical level. Tubulin Polymerization Promoting Protein (TPPP), as a prominent pathological partner of SYN, has been identified as a crucial factor in the Parkinson pathomechanism (Oláh J, Vic Norris V. Lehotzky A, Ovadi J Perspective Strategies for Interventions in Parkinsonism: Remedying the Neglected Role of TPPP Cells (2024) 13, 338. and references therein).

TPPP, a brain specific disordered protein, displays multiply functions. At physiological conditions it regulates the dynamism and stability of the microtubule (MT) network and controls the differentiation of the oligodendrocytes (OLGs) (Lehotzky A, Lau P, T 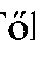 kés N, Muja N, Hudson LD, Ovádi J. Tubulin polymerization promoting protein (tppp/p25) is critical for oligodendrocyte differentiation Glia. (2010) 157-168. doi:10.1002/glia.20909.). At pathological conditions the brain specific TPPP promotes SYN assembly, and deposits of neurodegenerative diseases (Kovács GG, Gelpi E, Lehotzky A et al. The brain-specific protein TPPP/p25 in pathological protein deposits of neurodegenerative diseases Acta Neuropathol (2007) 113: 153-161)

Although SYN and TPPP are expressed in distinct cell types, dominantly in the neurons and OLGs, respectively; nevertheless, their hetero-assemblies are achieved in both cell types at pathological conditions by cell-to-cell transfer via the extracellular space (Oláh J, Lehotzky A, Szunyogh S, Szénási T, Ovádi J Microtubule-Associated Proteins with Regulatory Functions by Day and Pathological Potency at Night Cells 2020, 9, 357). The co-localization of the two marker proteins has been visualized by immunofluorescence confocal microscopy with specific antibodies in the brains of PD and MSA patients (Kovács GG, Lászlo L, Kovács J et al. Natively unfolded tubulin polymerization promoting protein TPPP/p25 is a common marker of alpha-synucleinopathies Neurobiology of Disease (2004) 17;155- 162). Indeed, most of the research related to Parkinsonism undeservedly neglects the role of TPPP, and its promotion of SYN aggregation (Oláh J and Ovádi J. Pharmacological targeting of a-synuclein and TPPP/p25 in Parkinson's disease: challenges and opportunities in a Nutshell FEBS Letters (2019) 593:1641).

### SUMMARY OF THE INVENTION

An agent for use in inhibiting the formation of or disintegrating the alpha-synuclein - alpha-synuclein (SYN-SYN) complex, wherein the agent inhibits or disrupts the association of tubulin polymerization promoting protein (TPPP) and SYN.

Preferably the agent directly inhibits or disrupts the association of SYN and TPPP.

Preferably the agent indirectly inhibits the formation of or disintegrates the SYN-SYN complex through inhibiting or disrupting the SYN-TPPP complex.

Preferably the agent inhibits or disrupts the association of tubulin polymerization promoting protein (TPPP) and SYN at the interface of the SYN-TPPP complex

Preferably the agent binds to or disrupts the amino acid sequence of SGVTK (SEQ ID NO 9) in TPPP, thereby inhibiting the formation of or disintegrating the SYN-TPPP complex.

Preferably the agent binds to the amino acid sequence of SGVTK (SEQ ID NO 9) in TPPP, thereby inhibiting the formation of or disintegrating the SYN-TPPP complex.

Preferably the agent binds to or disrupts the amino acid sequence of SEQ ID NO 1 in TPPP, thereby inhibiting the formation of or disintegrating the SYN-TPPP complex.

Preferably the agent binds to the amino acid sequence of SEQ ID NO 1 in TPPP, thereby inhibiting the formation of or disintegrating the SYN-TPPP complex.

Preferably the agent binds to or disrupts the amino acid sequence of SEQ ID NO 3 in TPPP, thereby inhibiting the formation of or disintegrating the SYN-TPPP complex.

Preferably the agent binds to the amino acid sequence of SEQ ID NO 3 in TPPP, thereby inhibiting the formation of or disintegrating the SYN-TPPP complex.

Preferably the agent binds to or disrupts the amino acid sequence of SEQ ID NO 8 in TPPP, thereby inhibiting the formation of or disintegrating the SYN-TPPP complex.

Preferably the agent binds to the amino acid sequence of SEQ ID NO 8 in TPPP, thereby inhibiting the formation of or disintegrating the SYN-TPPP complex.

Preferably the agent binds to or disrupts the amino acid sequence of SEQ ID NO 2 in SYN, thereby inhibiting the formation of or disintegrating the SYN-TPPP complex.

Preferably the agent binds to the amino acid sequence of SEQ ID NO 2 in SYN, thereby inhibiting the formation of or disintegrating the SYN-TPPP complex.

Preferably the agent binds to or disrupts the amino acid sequence of SEQ ID NO 4 in SYN, thereby inhibiting the formation of or disintegrating the SYN-TPPP complex.

Preferably the agent binds to the amino acid sequence of SEQ ID NO 4 in SYN, thereby inhibiting the formation of or disintegrating the SYN-TPPP complex.

Preferably the agent binds to or disrupts the amino acid sequence of SEQ ID NO 5 in SYN, thereby inhibiting the formation of or disintegrating the SYN-TPPP complex.

Preferably the agent binds to the amino acid sequence of SEQ ID NO 5 in SYN, thereby inhibiting the formation of or disintegrating the SYN-TPPP complex.

Preferably the agent comprises the amino acid sequence of SEQ ID NO 9 or an amino acid sequence having at least 60% sequence identity, preferably at least 80% sequence identity with SEQ ID NO 9.

Preferably the agent comprises the amino acid sequence of SEQ ID NO 1 or an amino acid sequence having at least 70% sequence identity, preferably at least 80% sequence identity, preferably at least 85% sequence identity, preferably at least 90% sequence identity, preferably at least 95% sequence identity with SEQ ID NO 1.

The agent for use according to claim 3, wherein the agent comprises the amino acid sequence of SEQ ID NO 1 or an amino acid sequence having at least 70% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 3.

Preferably the agent comprises the amino acid sequence of SEQ ID NO 8 or an amino acid sequence having at least 50% sequence identity, preferably at least 75% sequence identity, with SEQ ID NO 8.

Preferably the agent comprises the amino acid sequence of SEQ ID NO 2 or an amino acid sequence having at least 70% sequence identity, at least 75% sequence identity preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 2.

Preferably the agent comprises the amino acid sequence of SEQ ID NO 4 or an amino acid sequence having at least 70% sequence identity, at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 4.

Preferably the agent comprises the amino acid sequence of SEQ ID NO 5 or an amino acid sequence having at least 70% sequence identity, at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 5.

A combination of
an agent inhibiting the formation of or disrupting the association of SYN-TPPP and
an agent inhibiting the formation of or disrupting the association of SYN-SYN,
for use in the treatment of PD or DLB or MSA by inhibiting the formation of or disintegrating both the SYN-SYN and the SYN-TPPP associations.

Preferably the agent inhibiting the formation of or disrupting the association of SYN-TPPP inhibits or disrupts the association of SYN-TPPP at the interface of the SYN-TPPP complex.

Preferably the agent inhibiting the formation of or disrupting the association of SYN-TPPP directly inhibits or disrupts the association of SYN and TPPP.

Preferably the agent inhibiting the formation of or disrupting the association of SYN-TPPP binds to or disrupts the amino acid sequence of SGVTK (SEQ ID NO 9) in TPPP, thereby inhibiting the formation of or disintegrating the SYN-TPPP complex.

Preferably the agent inhibiting the formation of or disrupting the association of SYN-TPPP binds to the amino acid sequence of SGVTK (SEQ ID NO 9) in TPPP, thereby inhibiting the formation of or disintegrating the SYN-TPPP complex.

Preferably the agent inhibiting the formation of or disrupting the association of SYN-TPPP binds to or disrupts the amino acid sequence of SEQ ID NO 1 in TPPP, thereby inhibiting the formation of or disintegrating the SYN-TPPP complex.

Preferably the agent inhibiting the formation of or disrupting the association of SYN-TPPP binds to the amino acid sequence of SEQ ID NO 1 in TPPP, thereby inhibiting the formation of or disintegrating the SYN-TPPP complex.

Preferably the agent inhibiting the formation of or disrupting the association of SYN-TPPP binds to or disrupts the amino acid sequence of SEQ ID NO 3 in TPPP, thereby inhibiting the formation of or disintegrating the SYN-TPPP complex.

Preferably the agent inhibiting the formation of or disrupting the association of SYN-TPPP binds to the amino acid sequence of SEQ ID NO 3 in TPPP, thereby inhibiting the formation of or disintegrating the SYN-TPPP complex.

Preferably the agent inhibiting the formation of or disrupting the association of SYN-TPPP binds to or disrupts the amino acid sequence of SEQ ID NO 8 in TPPP, thereby inhibiting the formation of or disintegrating the SYN-TPPP complex.

Preferably the agent inhibiting the formation of or disrupting the association of SYN-TPPP binds to the amino acid sequence of SEQ ID NO 8 in TPPP, thereby inhibiting the formation of or disintegrating the SYN-TPPP complex.

Preferably the agent inhibiting the formation of or disrupting the association of SYN-TPPP binds to or disrupts the amino acid sequence of SEQ ID NO 2 in SYN, thereby inhibiting the formation of or disintegrating the SYN-TPPP complex.

Preferably the agent inhibiting the formation of or disrupting the association of SYN-TPPP binds to the amino acid sequence of SEQ ID NO 2 in SYN, thereby inhibiting the formation of or disintegrating the SYN-TPPP complex.

Preferably the agent inhibiting the formation of or disrupting the association of SYN-TPPP binds to or disrupts the amino acid sequence of SEQ ID NO 4 in SYN, thereby inhibiting the formation of or disintegrating the SYN-TPPP complex.

Preferably the agent inhibiting the formation of or disrupting the association of SYN-TPPP binds to the amino acid sequence of SEQ ID NO 4 in SYN, thereby inhibiting the formation of or disintegrating the SYN-TPPP complex.

Preferably the agent inhibiting the formation of or disrupting the association of SYN-TPPP binds to or disrupts the amino acid sequence of SEQ ID NO 5 in SYN, thereby inhibiting the formation of or disintegrating the SYN-TPPP complex.

Preferably the agent inhibiting the formation of or disrupting the association of SYN-TPPP binds to the amino acid sequence of SEQ ID NO 5 in SYN, thereby inhibiting the formation of or disintegrating the SYN-TPPP complex.

Preferably the agent inhibiting the formation of or disrupting the association of SYN-TPPP comprises the amino acid sequence of SEQ ID NO 9 or an amino acid sequence having at least 60% sequence identity, preferably at least 80% sequence identity with SEQ ID NO 9.

Preferably the agent inhibiting the formation of or disrupting the association of SYN-TPPP comprises the amino acid sequence of SEQ ID NO 1 or an amino acid sequence having at least 70% sequence identity, preferably at least 80% sequence identity, preferably at least 85% sequence identity, preferably at least 90% sequence identity, preferably at least 95% sequence identity with SEQ ID NO 1.

The agent for use according to claim 3, wherein the agent comprises the amino acid sequence of SEQ ID NO 1 or an amino acid sequence having at least 70% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 3.

Preferably the agent inhibiting the formation of or disrupting the association of SYN-TPPP comprises the amino acid sequence of SEQ ID NO 8 or an amino acid sequence having at least 50% sequence identity, preferably at least 75% sequence identity, with SEQ ID NO 8.

Preferably the agent inhibiting the formation of or disrupting the association of SYN-TPPP comprises the amino acid sequence of SEQ ID NO 2 or an amino acid sequence having at least 70% sequence identity, at least 75% sequence identity preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 2.

Preferably the agent inhibiting the formation of or disrupting the association of SYN-TPPP comprises the amino acid sequence of SEQ ID NO 4 or an amino acid sequence having at least 70% sequence identity, at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 4.

Preferably the agent inhibiting the formation of or disrupting the association of SYN-TPPP comprises the amino acid sequence of SEQ ID NO 5 or an amino acid sequence having at least 70% sequence identity, at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 5.

Preferably one of the agents is 2-hydroxy-5-nitro-6-(3-nitrophenyl)-4-(trifluoromethyl)nicotinonitrile (SC-D). Preferably the agent inhibiting the formation of or disrupting the association of SYN-SYN is SC-D.

Preferably SC-D and the agent inhibiting the formation of or disrupting the association of SYN-TPPP are administered in a dose providing mutual effect.

### BRIED DESCRIPTION OF THE FIGURES

Figure 1A and B. SYN mutants can bind to TPPP; no significant difference can be detected; however, the competitive effects of some mutant SYN forms are different; a prominent decrease is caused by S87E SYN, which is comparable with that of the control (wild SYN).
Figure 2 The effect of the mutant SYNs on the self-association of the wild SYN as detected by BiFC technology.
Figure 3. Segments of TPPP selected to study their potential role in the inhibition of SYN-SYN associations.
Figure 4. Inhibition of TPPP fragments on the SYN-SYN association.
Figure 5. Intracellular aggregations are formed in the presence of both SYN and TPPP.
Figure 6. The hetero-association of the SYN with TPPP is formed at the expense of the self-assembly of SYN.
Figure 7. Inhibitory potency of SC-D and/or SYN fragment detected at Tf-T assay. Conditions: 100 µM SYN, 25 µM TPPP, 100 µM SC-D, 37°C.
Figure 8. SYN in the cells with or without TPPP as detected by cELISA
Figure 9. Effects of commercial SYN-SYN inhibitors and peptide fragments on the hetero-association of SYN with TPPP. S15: SYN126-140, SEQ ID NO 2; BA75: SEQ ID NO 1; BA74: SEQ ID NO 10.
Figure 10. Quantification of the effective fragments on the SYN-TPPP association.

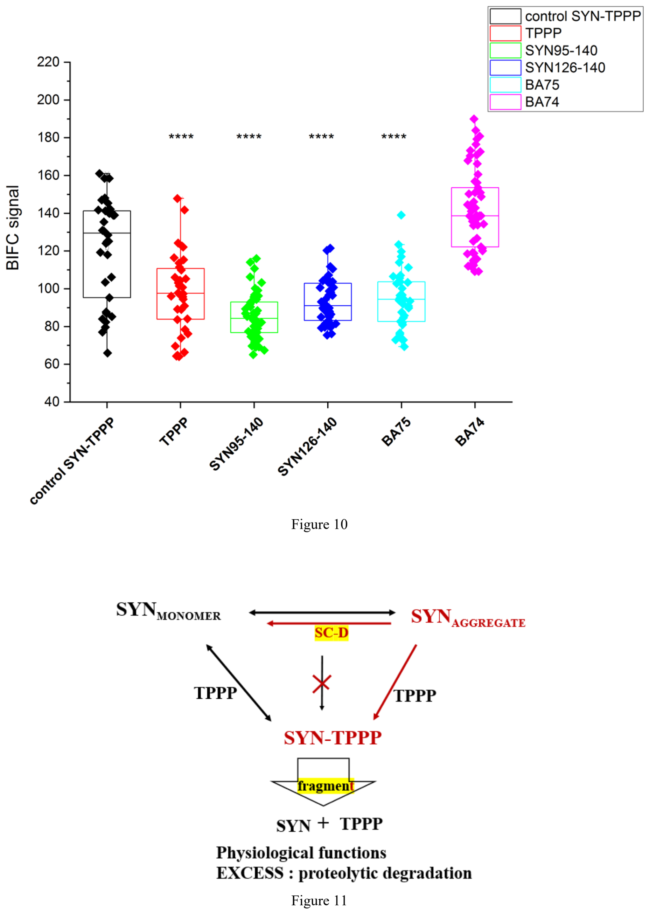

Figure 11. Inhibition of the homo- and hetero-assembly of SYN by fragments with or without SC-D. Schematic illustration of the mode of action of a combination of two agents for use in the treatment of a synucleinopathy, by inhibiting the formation of or disintegrating both the SYN-SYN and the SYN-TPPP associations. One of the agents is an agent (e.g. SC-D) capable of inhibiting the formation of or disrupting the association of SYN-SYN and the other agent (e.g. S-frag-15, SYN126-140, SEQ ID NO 2, aa 126-140 of the human α-synuclein) is capable of inhibiting the formation of or disrupting the association of SYN-TPPP.
Figure 12. A: Pair-wise interaction of the immobilized SIRT2 with tubulin and/or TPPP:ELISA. B: Association of SIRT2-TPPP complex on MT network: BiFC coupled with IF microscopy.
Figure 13. Molecular mechanism of the TPPP-derived deacetylase inhibition.
Figure 14. Mutual anti-aggregative effects of the interface-targeting SYN fragments and TPPP as a deacetylation inhibitor.

### DETAILED DESCRIPTION OF THE INVENTION

The terms "human TPPP protein" and "TPPP" refer to the human tubulin polymerization promoting protein having the sequence of SEQ ID NO 6.

The terms "human α-synuclein protein", "synuclein" and "SYN" refer to the human α-synuclein protein having the sequence of SEQ ID NO 7.

A "treatment" refers to any process, action, application, therapy, or the like, wherein the subject or patient is under aid, in particular medical or veterinarian aid with the object of improving the subjects's or patient's condition, either directly or indirectly. Improving the subjects's condition may include improving an aesthetic condition (cosmetic treatment) and/or may include, in particular, restoring or maintaining normal function of an organ or tissue, preferably at least partly restoring or maintaining health (medical or veterinarian treatment). Treatment typically refers to the administration of an effective amount of a compound or composition described herein. Treatment may relate to or include medical or veterinarian treatment and cosmetic treatment, in particular medical or veterinarian treatment.

"Preventing" or "prevention" of the development of a disease or condition refers to at least the reduction of likelihood of the risk of or susceptibility to acquiring a disease or disorder, or preferably causing at least one of the clinical symptoms of the disease or disorder not to develop in a patient that may be exposed to or predisposed to the disease but does not yet experience or display symptoms of the disease.

Although the cause of most cases of PD is unknown, about 10 % of the PD cases are due to the mutations of SNCA encoding SYN, which usually lead to early-onset PD. PD-causing A53T SYN mutation elevates SYN expression and compromises autophagy in human neurons and in the CNS of zebrafish and mice (Son SM, Siddiqi FH, Lopez et al. Alpha-synuclein mutations mislocalize cytoplasmic p300 compromising autophagy, which is rescued by ACLY inhibition

Cell press (2025) 18;113:1908-1924.e13). Missense mutations in the gene encoding SYN are associated with familial Parkinson's disease, which affect the aggregation of the protein (Flagmeier P , Meisl G, Vendruscolo M et al. Mutations associated with familial Parkinson's disease alter the initiation and amplification steps of α-synuclein aggregation PNAS (2016) 113:37). Mutations of SYN identified in PD patients so far are: A30P, E46K, G51D, A53T, S87E, S129A, and S129D. The influence of these SYN mutations on their interactions with TPPP was studied at molecular and cellular levels. The SYN mutants were produced by recombinant technology, and the proteins were taken up from the medium to mimic the pathological conditions as described previously (Lehotzky A, Oláh J, Fekete JT, Szénási T et al, Co-Transmission of Alpha-Synuclein and TPPP/p25 Inhibits Their Proteolytic Degradation in Human Cell Models

Frontiers in Molecular Biosciences (2021) 8:666026 ) (cf. Examples).

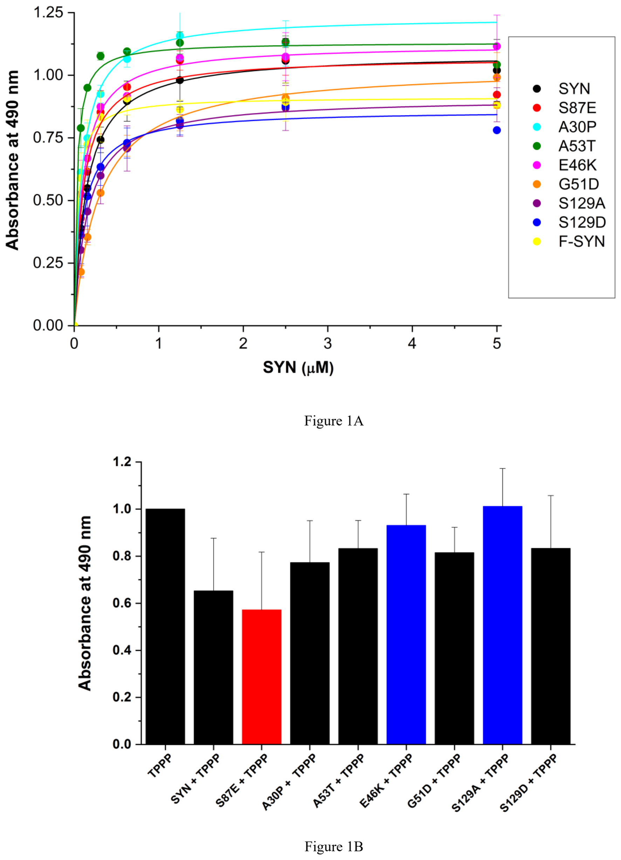

As shown in Fig. 1A, the SYN mutants can bind to TPPP; no significant difference can be detected; however, the competitive effects of some mutant SYN forms are different; a prominent decrease is caused by S87E SYN, which is comparable with that of the control (wild SYN) (Fig. 1B). In addition, the experimental finding with living human cells expressed mutant SYN showed the co-localizations of TPPP and SYN. These data suggest that the mutant segments of SYN are not involved, at least not significantly, in the hetero-association of SYN and TPPP. The influence of the SYN mutations on the self-assembly was also revealed by Bifunctional Fluorescence Complementation (BiFC) technology, and the BiFC signal was detected by confocal fluorescence microscopy.

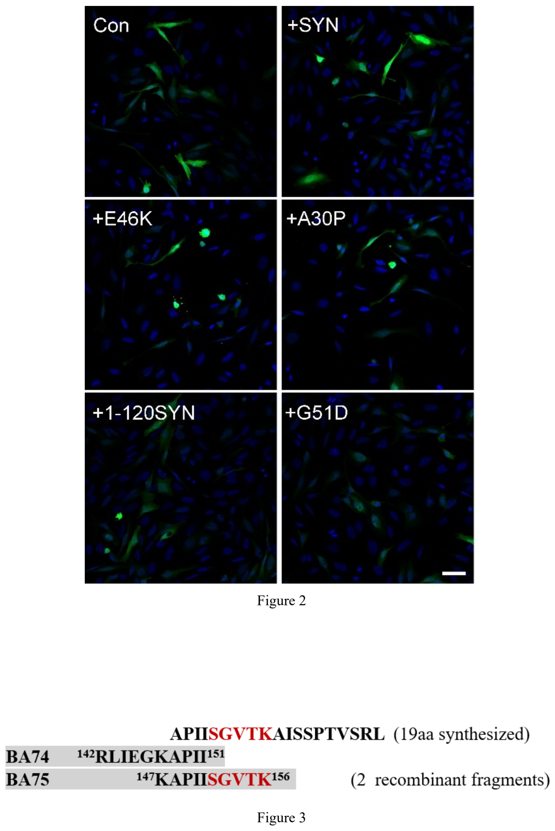

Fig. 2 shows that the G51D SYN mutation, in contrast to the others, inhibits the SYN-SYN association (no BiFC signal). Since earlier data provided evidence for the involvement of the C-terminus of SYN in its hetero-association with TPPP (Szunyogh S, Oláh J, Szénási T, Szabó A, Ovádi J. Targeting the interface of the pathological complex of α-synuclein and TPPP/p25. Biochim Biophys Acta. (2015) 1852:2653-61) it can be concluded that distinct segments of the SYN take part in the homo- and hetero-associations.

The mutual occurrence/accumulation of SYN and the presence of TPPP leads to PD via the formation of the fatal oligomers. This pathological situation provides a unique opportunity for the development of an interface drug target; at the same time, it is challenging (Oláh J, Lehotzky A, Szunyogh S, Szénási T, Ovádi J. Microtubule-Associated Proteins with Regulatory Functions by Day and Pathological Potency at Night. Cells 2020, 9, 357). Different segments of the TPPP were synthetized or produced by recombinant technology to test their inhibitory potency against the SYN assembly, since clear evidence was obtained for the key role of the CORE region (45-175 aa) of the TPPP. The segments of TPPP selected to study their potential role in the inhibition of SYN-SYN associations are shown on Figure 3.

The interface of the SYN-TPPP as a potential drug target was identified in molecular studies using recombinant proteins. The identified/validated fragments, which inhibit specifically the interaction of SYN with TPPP, are the following: SYN127-140, SYN95-140 and TPPP145-156 as demonstrated by different ELISA assays, immunofluorescence confocal microscopy coupled with or without BiFC technology using human recombinant proteins and HeLa and SH-SY5Y human cell lines (Szunyogh et al., 2015, supra; Szénási T, Oláh J, Szabó A, Szunyogh S, Láng A, Perczel A, Lehotzky A, Uversky VN, Ovádi J. Challenging drug target for Parkinson's disease: Pathological complex of the chameleon TPPP/p25 and alpha-synuclein proteins Affiliations. Biochim Biophys Acta Mol Basis Dis (2017) 63:310-323).

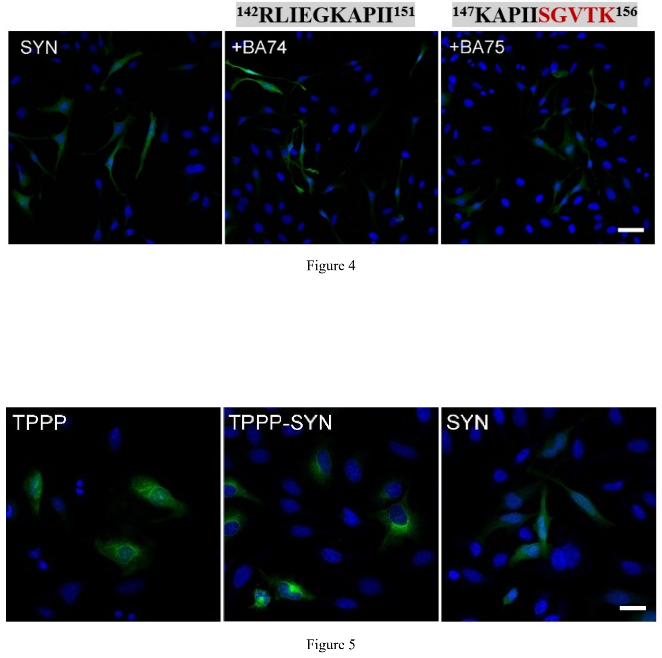

Nevertheless, recent research data reveals stronger interacting potency for the association of SYN -TPPP than that of SYN-SYN. Figure 5 illustrates that at same experimental conditions aggregates can be visualized exclusively at the intracellular presence of both proteins.

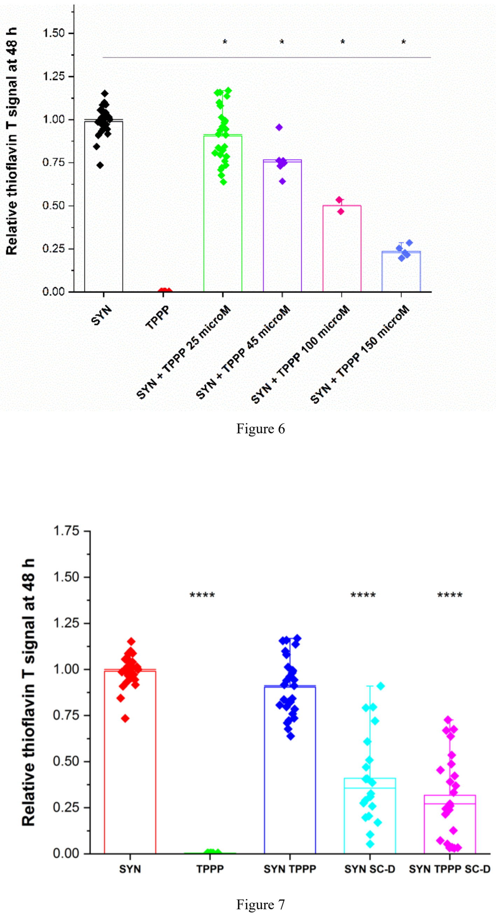

The SYN-SYN interaction is influenced by TPPP in a concentration dependent manner due to the firm binding of TPPP to SYN (Kd ~100 nM). The modifying effect of TPPP on the self-assembly of the SYN was established by Thioflavin-T (Tf-T) assay (Figure 6). The SYN assembly, as a relevant pathological structure, has been recently used as a biomarker of PD (Siderowf A, Concha-Marambio L, Lafontan D-Et et al. Assessment of heterogeneity among participants in the Parkinson's Progression Markers Initiative cohort using α-synuclein seed amplification: a cross-sectional study. Lancet Neurol (2023) 22:407-417. doi: 10.1016/S1474-4422(23)00109-6.). A technology developed recently to follow SYN assembly was applied to test and quantify the effects of TPPP on the self-assembly of SYN in vitro by Thioflavin-T assay.

Thioflavin T (Tf-T) is a cationic Benzothiazole dye that shows enhanced fluorescence upon binding to amyloid structures. This recently introduced cellular assay (αSyn-SAA assay) (Siderowf et al 2023, supra) was applied to detect/characterized the effects of the interacting partners on the toxic SYN structures.

As shown in Figure 6, TPPP reduces the SYN self-assembly in a concentration dependent manner due to the interrelated connection between SYN-SYN and SYN-TPPP association and the firm binding of TPPP to SYN. At higher concentrations, less Tf-T signals can be detected indicating that the presence of TPPP shifts the self-assembly of SYN-SYN towards the formation of SYN-TPPP.

The effect of SC-D, a known inhibitor of SYN aggregation (Peña-Díaz P,1 Pujols J, Vasili E, Pinheirol F et al. The small aromatic compound SynuClean-D inhibits the aggregation and seeded polymerization of multiple α-synuclein strains. J Biol Chem (2022) 298:101902.), was also tested by Tf-T assay (Figure 7). As expected, it significantly decreased the SYN aggregation. A further decrease was observed in the presence of TPPP due to the mutual effects of SC-D and TPPP on SYN assembly.

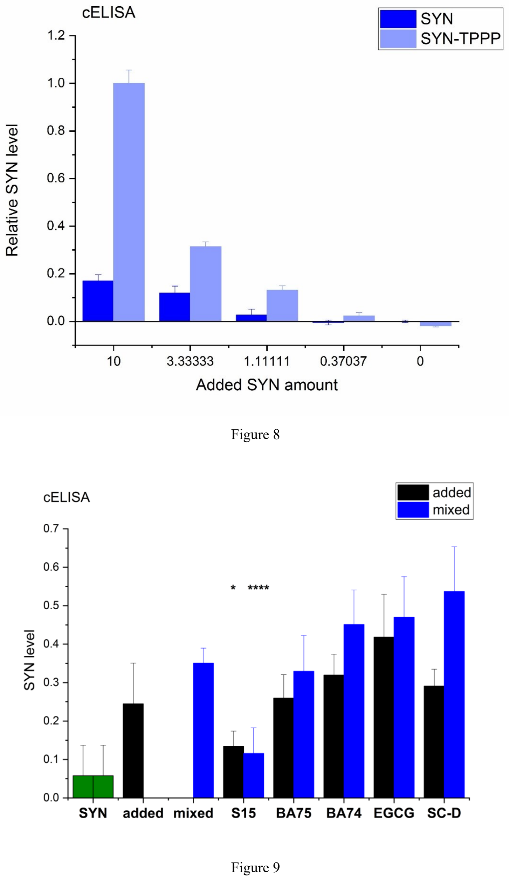

It is an important finding that the self-assembly of SYN is reduced by TPPP in a concentration-dependent manner. cELISA experiment was performed, when different amounts of SYN or SYN-TPPP mix were added to the cells, then SYN was detected by a specific antibody. As shown in Figure 8, the SYN signal is concentration-dependent, as expected. Importantly, the presence of TPPP significantly increased the detected amount of SYN indicating that SYN cannot be degraded by the cells in the presence of TPPP. The autophagy degradation of the assembled SYN-TPPP is impeded due to the hindrance of the autophagy maturation at the stage of LC3B - SQSTM1/p62-derived autophagosome formation and its fusion with lysosome. This observation clearly indicates that the SYN homo- and hetero-assemblies should be targeted, since SYN liberated from its association with TPPP can be proteolytically degraded.

The SYN and TPPP are taken up by the HeLa cells from the medium to mimic the in vivo situation when SYN and TPPP expressed endogenously in neuron and OLG, respectively, and are transferred via extracellular space. The uptake of the proteins was carried out in two different manners: premixed or added sequentially. The level of the SYN was determined by its association with TPPP, the formation of which counteracts the proteolytic degradation, but not the free ones. In the control samples (no TPPP), no significant difference was in the SYN level applying distinct uptake. As illustrated in Figure 9, while the SYN fragment significantly inhibits the hetero-association of the SYN with TPPP due to its anti-aggregative potency; the commercial agents, inhibitors of the self-assembly of the SYN (EGCG and SC-D) display no inhibitory influence on the hetero-association of SYN.

The quantitative effects of the fragments were revealed by immunofluorescent microscopy coupled with BiFC technology using Venus's pair labelling. The comparison of the SYN and TPPP fragments, in agreement with the earlier data obtained by human recombinant proteins, revealed similar inhibitory potency of BA75 and the two SYN fragments against the hetero-association of SYN and TPPP. As shown in Figure 10, in the experiment performed with living human cells, the BiFC signal of the SYN-TPPP associations are reduced by addition of SYN and TPPP fragments, supporting the data obtained by either recombinant proteins or at cellular level.

These results provide evidence for the role of these fragment agents in the destruction of both homo- and hetero-association of the SYN. This knowledge allows the evaluation of an "EASY-USED" method to screen the impact of agents for their inhibitory potency (anti-aggregative effect); this is the competitive ELISA. SYN or TPPP is immobilized on the plate, then TPPP + agent or SYN + agent is added, respectively, and the inhibitory effect of agents can be visualized by specific antibody (Szabó A, Oláh J, Szunyogh S, Lehotzky A. et al. Modulation Of Microtubule Acetylation By the Interplay Of TPPP/p25, SIRT2 And New Anticancer Agents With Anti-SIRT2 Potency Sci Rep (201) 7:17070.).

Conclusion: TPPP promotes SYN association/aggregation at the expense for the SYN self-assembly. Their destruction by fragment agents, which process is prompted (not necessarily) by the presence of SC-D compound, leads to the degradation of the excess disassembled SYN and the recovery of their physiological functions (Figure 11).

The innovative strategy introduced in this intervention refers to the disassembly/destruction of the SYN-TPPP association by the interface-targeting peptide agents (SYN or TPPP fragments), which do destruct the SYN assembly due to the shift of the equilibrium between the homo- and hetero-assembly of SYN. The enriched monomer SYNs produced by the destruction/inhibition of SYN associations are prey of proteolytic degradation such as autophagy and proteasome machinery. The novelty of this invention is not only to reveal the role of TPPP in the assembly of SYN at the expense of the SYN self-assembly, but to offer solution for the unknown situations occurring in the pathological brain inclusions, namely, the unknown distribution/ratio of the homo- and hetero complexes of SYN.

TPPP, as Janus-faced protein controls the acetylation

Janus-faced TPPP displays multiple activity: on one hand, it promotes SYN assembly, on the other hand, it counteracts deacetylation of the MT network by its inhibition of Sirtuin2 (SIRT2). At physiological conditions, TPPP increases the acetylation level of the MT network by displaying powerful impeding potency against the intracellular deacetylation. The increased acetylation due to the inhibition of SIRT2 is derived by the indirect (piggy-back) binding of the SIRT2 deacetylase to the MT network via TPPP. Indeed, the co-localization of the SIRT2-TPPP complex on the MT network was visualized by immunofluorescence microscopy (Szabó et al. 2017, supra).

Several studies have revealed the influences of the PTMs on both physiological and pathological functions of proteins (Hassanzadeh K, Liu J, Maddila S, Mouradian MM. Posttranslational Modifications of α-Synuclein, Their Therapeutic Potential, and Crosstalk in Health and Neurodegenerative Diseases. Pharmacol Rev 76:1254-1290, 2024). The acetylation of proteins is one of the most prevalent PTMs in eukaryotes, which is controlled by histone acetyltransferases and histone deacetylases. SIRT2, a predominant deacetylase in the cytoplasm of the human brain is an important factor in the control of the dynamics and stability of MT. The overproduction of SIRT2 causes MT instability as well as neuronal toxicity. However, the inhibition of SIRT2 can at least partly prevent the apoptotic cell death concomitant with the increased number of intracellular inclusions visualized in cellular models of PD/MSA (Harting K and Knöll B.

SIRT2-mediated protein deacetylation: An emerging key regulator in brain physiology and pathology. Eur J Cell Biol (2010) 89:262-9.).

At physiological conditions SYN is acetylated on lysine 6 and 10 (Singha S, Khayachib A, Milnerwoodb AJ, DeMarcoa ML. Quantitative Profiling of Synuclein Species: Application to Transgenic Mouse Models of Parkinson's Disease. Journal of Parkinson's Disease (2020) 10:613-621). Genetic manipulation of SIRT2 levels in vitro and in vivo modulates the levels of SYN acetylation and its aggregation. The deacetylation of these lysine residues by SIRT2 is a key destructive mechanism resulting in SYN aggregation and toxicity (Oliveira RM, Miranda HV, R et al. The mechanism of sirtuin 2-mediated exacerbation of alpha-synuclein toxicity in models of Parkinson disease. PLoS Biol (2017) 15: e2000374). In knockout SIRT2 mice, no autophagy dysfunction was detected after the administration of the neurotoxin MPTP, suggesting the important role of SIRT2 in these processes (Patel D, Soni R, Shah J. Decoding the Role of Nuclear Sirtuins in Parkinson's Pathogenesis. ACS Chem. Neurosci. (2024) 15, 3615-3625). In addition, the Lys acetylation of SYN was confirmed by animal experiments (C57BL/6J mice, AAV-h SYN), which resulted in enhanced acetylation level of SYN as detected by immunofluorescence microscopy using specific SYN and acetyl Lys antibodies. (Brembati V, Faustini G, Longhena F et al. Changes in α-Synuclein Posttranslational Modifications in an AAV-Based Mouse Model of Parkinson's Disease. Int. J. Mol. Sci. (2023) 24, 13435).

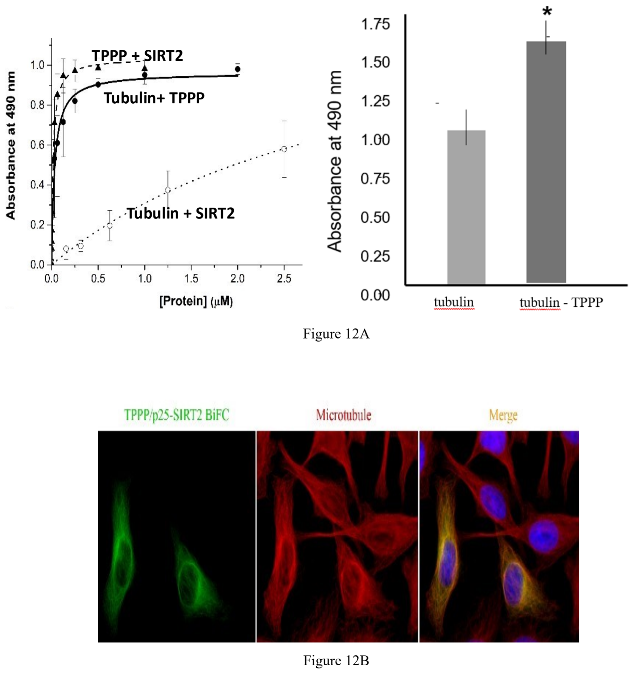

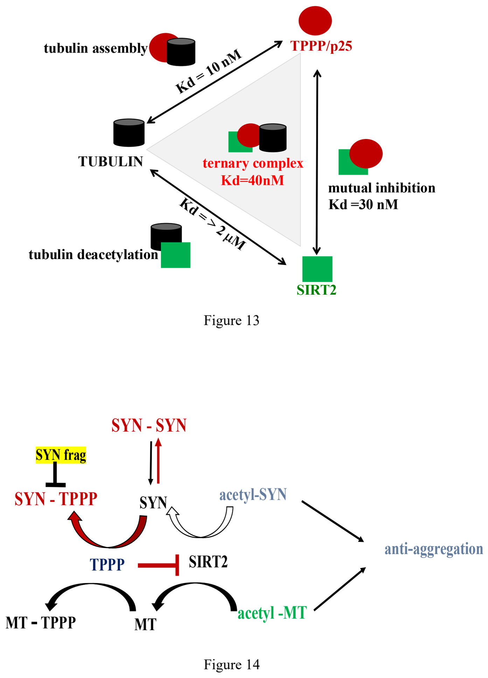

The intracellular interaction between SIRT2 and TPPP as well as the localization of their complex on the MT network was demonstrated by BiFC using Venus labelled protein pairs. The BiFC complex of TPPP and SIRT2 was visualized by confocal immunofluorescence microscopy (Figure 12) (Szabó et al 2017, supra). The action of TPPP-derived inhibition of SIRT2 deacetylase plays central role in the control of the acetylation level of the tubulin (Tökési N, Horváth I, Szabó B, Oláh J. et al. TPPP/p25 promotes tubulin acetylation by inhibiting histone deacetylase 6. J Biol Chem (2010);285(23):17896-906). Inhibition of SIRT2 affects SYN-mediated cytotoxicity, which displays a neuroprotective role. The association of SIRT2 with TPPP leads to the stabilization of the MT network, which promotes MT-dependent transportation of SYN into the inclusion (Hasegawa T, Baba T, Kobayashi M et al. Role of TPPP/p25 on a-synuclein-mediated oligodendroglial degeneration and the protective effect of SIRT2 inhibition in a cellular model of multiple system atrophy. Neurochemistry International (2010) 2719:10). Consequently, the molecular modulation of MT dynamics/stability seems to be an effective way, which can counteract neurodegenerative aggregations. The mechanism of the TPPP-derived SIRT2 inhibition is based upon the "indirect interaction" of SIRT2 to tubulin under intracellular conditions (Figure 13).

Characteristic feature of PD is the toxic inclusions in the neurons formed by the pathologically accumulated SYN (SYN seed) and the transmission of the TPPP from the OLGs. The SYN-TPPP association is formed at the expense of the SYN self-assembly. Specific fragment agents SYN127-140, SYN95-140 or TPPP 145-156 destruct the SYN-TPPP association resulting in recovery of the physiological functions of the partner proteins (Figure 14).

The association of the TPPP with SIRT2 inhibits deacetylation by piggy-back mechanism leading to anti-aggregative effect: the acetylation of SYN counteracts its assembly: the acetylated MT network transports a small fatal aggregate to the inclusions. In conclusion, the Janus-faced TPPP by promoting SYN assembly and inhibiting the deacetylation in cooperation with the fragment agent(s), can provide an opportunity to cure PD.

The invention is based on the finding that TPPP promotes SYN aggregation due to their interactions, which is formed at the expense of SYN-SYN association. The destruction of the SYN-TPPP interaction by an agent could lead to the pathological enrichment of uncomplexed SYNs, however, they fall victim to the proteolytic digestive systems. Treatment is based on the finding that by inhibiting the formation of SYN-SYN aggregates through the inhibition of the formation of SYN-TPPP aggregates, both SYN and TPPP may be degraded.

The interface of the SYN-TPPP interaction as potential drug target was identified. Its inhibition can be specifically performed by peptide fragments of the partner proteins: SYN127-140, SYN95-140 and TPPP145-156 as demonstrated by different ELISA assays, immunofluorescence confocal microscopy coupled with or without Bifunctional Fluorescence Complementation technology using human recombinant proteins and human living cells (Szunyogh et al., 2015 supra; Szénási et al., 2017 supra; Oláh et al., 2020 supra).

An aspect of the invention is based on the finding that TPPP ensures that the equilibrium between the homo- and hetero-assembly of SYN (SYN-SYN and SYN-TPPP) is shifted towards the formation of the SYN-TPPP complex. This, in turn, leads to the reduction of the SYN-SYN association. Consequently, inhibition of the SYN-TPPP assembly results in the inhibition (or degradation) of both pathological assemblies, namely SYN-SYN and SYN-TPPP (Figure 3).

The association of TPPP with SYN is formed at the expense of SYN-SYN association. SYN deliberated from its association with TPPP is proteolytically degraded resulting in uncomplexed TPPP.

This finding allows us to target both types of pathological aggregation involved in synucleinopathies at a single point: the interface of SYN-TPPP of the pathological complex of SYN and TPPP. Inhibition of the formation and/or disintegration of both the associations of SYN may be achieved by one agent, irrespective of the ratio of homo-association to hetero-association.

The strategy also renders the recovery of the physiological function of both hallmark proteins possible; in addition, it eliminates the excess SYN accumulated at pathological conditions by proteolytic degradation (Figure 15).

The interface of the pathological complex of SYN and TPPP has been characterised by Szénási et. al. (Challenging drug target for Parkinson's disease: Pathological complex of the chameleon TPPP/p25 and alpha-synuclein proteins. Biochim Biophys Acta Mol Basis Dis. 2017 Jan;1863(1):310-323) and Szunyogh et al. (Targeting the interface of the pathological complex of alpha-synuclein and TPPP/p25. Biochim Biophys Acta. 2015 Dec;1852(12):2653-61). Briefly, it was found that the core region of (human) TPPP (amino acids 44-174: GAAASPELSA LEEAFRRFAV HGDARATGRE MHGKNWSKLC KDCQVIDGRN VTVTDVDIVF SKIKGKSCRT ITFEQFQEAL EELAKKRFKD KSSEEAVREV HRLIEGKAPI ISGVTKAISS PTVSRLTDTT KFTG) is involved in the formation of the pathological SYN-TPPP associations, while the site involved in the binding of TPPP to tubulin is elsewhere in the protein. Within the core region, aminos acids 147-156 (KAPIISGVTK) and 59-62 (AVHG) of the human TPPP protein (MADKAKPAKA ANRTPPKSPG DPSKDRAAKR LSLESEGAGE GAAASPELSA LEEAFRRFAV HGDARATGRE MHGKNWSKLC KDCQVIDGRN VTVTDVDIVF SKIKGKSCRT ITFEQFQEAL EELAKKRFKD KSSEEAVREV HRLIEGKAPI ISGVTKAISS PTVSRLTDTT KFTGSHKERF DPSGKGKGKA GRVDLVDESG YVSGYKHAGT YDQKVQGGK) were found to form the interface between SYN and TPPP.

Szunyogh et al (supra) found that amino acids 95-140 (KKDHM GKGEEGASQE
GILEDMPVDP DNEAYEMPSE EGYQDYEPEA), 121-140 (DNEAYEMPSE EGYQDYEPEA) and 126-140 (EMPSE EGYQDYEPEA) but not 1-120 of SYN are involved in the formation of the pathological SYN-TPPP associations.

The above authors suggested that targerting the interface of the pathological SYN-TPPP complex to disintegrate the SYN-TPPP complex might be a new direction in anti-Parkinson drug development, however, did not realize that the disintegration or inhibition of the formation of the hetero-association of SYN can lead to the disintergration or inhibition of both the homo-association of SYN and the hetero-association of SYN.

In this aspect an agent inhibiting the association (binding) of TPPP and SYN at the interface of the SYN-TPPP complex is provided for use in the treatment of a synucleinopathy, preferably in the treatment of Parkinsonism, preferably in the treatment of PD or DLB or MSA by inhibiting the formation of or disintegrating the pathological SYN-SYN complex.

The invention is based on the finding that TPPP promotes SYN aggregation due to their interaction; the assembly of both SYN-SYN and SYN-TPPP can be reduced due to the destruction of the SYN-TPPP interaction by agents targeting or mimicking the binding interface of the SYN-TPPP complex.

An agent for use in the treatment of a synucleinopathy by inhibiting the formation of or disintegrating the SYN-SYN complex/interaction and/or SYN-TPPP complex/interaction is provided.

An agent inhibiting or disrupting the association (binding) of TPPP and SYN at the interface of the SYN-TPPP complex is provided for use in inhibiting the formation of or disintegrating the SYN-SYN complex.

A method for inhibiting the formation of or disintegrating the SYN-SYN complex in a subject in need of inhibiting the formation of or disintegrating the SYN-SYN complex is provided, wherein the method comprises administering a therapeutically effective dose of an agent capable of inhibiting or disrupting the association (binding) of TPPP and SYN at the interface of the SYN-TPPP complex.

A screening method for an agent for use in the treatment of a synucleinopathy by inhibiting the formation of or disintegrating the SYN-SYN complex is provided, wherein candidate agents are tested for their ability to inhibit the formation of the SYN-TPPP complex at the binding interface between SYN and TPPP or their ability to disintegrate the SYN-TPPP complex at the binding interface between SYN and TPPP.

A peptide for use in inhibiting the formation of or disintegrating the SYN-SYN complex is provided, wherein the peptide comprises the amino acid sequence of SEQ ID NO 8 (AVHG, aa 59-62 of the human TPPP protein).

A method for inhibiting the formation of or disintegrating the SYN-SYN complex in a subject in need of inhibiting the formation of or disintegrating the SYN-SYN complex is provided, wherein the method comprises administering a therapeutically effective dose of a peptide comprising the amino acid sequence of SEQ ID NO 8 to the subject.

A peptide for use in inhibiting the formation of or disintegrating the SYN-SYN complex is provided, wherein the peptide comprises the amino acid sequence of SEQ ID NO 1 (KAPIISGVTK; aa 147-156 of the human TPPP protein) or an amino acid sequence having at least 70%, at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 1.

A method for inhibiting the formation of or disintegrating the SYN-SYN complex in a subject in need of inhibiting the formation of or disintegrating the SYN-SYN complex is provided, wherein the method comprises administering a therapeutically effective dose of a peptide comprising the amino acid sequence of SEQ ID NO 1 (KAPIISGVTK; aa 147-156 of the human TPPP protein) or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 1, to the subject.

A peptide for use in inhibiting the formation of or disintegrating the SYN-SYN complex is provided, wherein the peptide comprises the amino acid sequence of SEQ ID NO 1 (KAPIISGVTK; aa 147-156 of the human TPPP protein) or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 1 and the amino acid sequence of SEQ ID NO 8.

A method for inhibiting the formation of or disintegrating the SYN-SYN complex in a subject in need of inhibiting the formation of or disintegrating the SYN-SYN complex is provided, wherein the method comprises administering a therapeutically effective dose of a peptide comprising the amino acid sequence of SEQ ID NO 1 (KAPIISGVTK; aa 147-156 of the human TPPP protein) or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 1 and the amino acid sequence of SEQ ID NO 8, to the subject.

A peptide for use in inhibiting the formation of or disintegrating the SYN-SYN complex is provided, wherein the peptide comprises the amino acid sequence of SEQ ID NO 2 (EMPSEEGYQDYEPEA; aa 126-140 of the human α-synuclein protein) or an amino acid sequence having at least 70%, at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 2.

A method for inhibiting the formation of or disintegrating the SYN-SYN complex in a subject in need of inhibiting the formation of or disintegrating the SYN-SYN complex is provided, wherein the method comprises administering a therapeutically effective dose of a peptide comprising the amino acid sequence of SEQ ID NO 2 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 2, to the subject.

Preferably the peptide for use or the peptide comprises the amino acid sequence of SEQ ID NO 3 (ASPELSA LEEAFRRFAV HGDARATGRE MHGKNWSKLC KDCQVIDGRN VTVTDVDIVF SKIKGKSCRT ITFEQFQEAL EELAKKRFKD KSSEEAVREV
HRLIEGKAPI ISGVTKAISS PTVSRLTDTT KFTG; aa 44-174 (core region) of the human TPPP protein) or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 2.

Preferably the peptide for use or the peptide comprises the amino acid sequence of SEQ ID NO 4 (DNEAYEMPSE EGYQDYEPEA; aa 121-140 of the human α-synuclein protein) or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 4.

Preferably the peptide for use comprises the amino acid sequence of SEQ ID NO 5 (KKDHM GKGEEGASQE GILEDMPVDP DNEAYEMPSE EGYQDYEPEA; aa 95-140 of the human α-synuclein protein) or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 5.

Preferably the agent or peptide for use in inhibiting the formation of or disintegrating the SYN-SYN complex is an agent or peptide for use in the treatment of a synucleinopathy, preferably Parkinsonism, preferably PD or DLB or MSA. Preferably the agent or peptide for use in inhibiting the formation of or disintegrating the SYN-SYN complex is an agent or peptide for use in the treatment of a synucleinopathy, preferably Parkinsonism, preferably PD or DLB or MSA by inhibiting the formation of or disintegrating the SYN-SYN complex. Preferably the agent or peptide for use in inhibiting the formation of or disintegrating the SYN-SYN complex is an agent or peptide for use in the treatment of a synucleinopathy, preferably Parkinsonism, preferably PD or DLB or MSA by inhibiting the formation of or disintegrating both the SYN-SYN and the SYN-TPPP complex.

Preferably the method for inhibiting the formation of or disintegrating the SYN-SYN complex is a method for the treatment of a synucleinopathy, preferably Parkinsonism, preferably PD or DLB or MSA. Preferably the method for the treatment of a synucleinopathy, preferably Parkinsonism, preferably PD or DLB or MSA is a method of treatment of a synucleinopathy, preferably Parkinsonism, preferably PD or DLB or MSA by inhibiting the formation of or disintegrating the SYN-SYN complex. Preferably the method for the treatment of a synucleinopathy, preferably Parkinsonism, preferably PD or DLB or MSA is a method of treatment of a synucleinopathy, preferably Parkinsonism, preferably PD or DLB or MSA by inhibiting the formation of or disintegrating both the SYN-SYN and the SYN-TPPP complex.

Preferably the peptide is up to 750 amino acid(aa) long, more preferably up to 500 aa long, more preferably up to 400 aa or up to 300 aa long.

Preferably the agent (for use) or the peptide (for use) is an agent or peptide capable of binding to the ¹⁵²SGVTK¹⁵⁶ (SEQ ID No 9) sequence of TPPP, thereby inhibiting the formation of or disintegrating the SYN-TPPP complex. Preferably the the peptide (for use) compises SEQ ID No 9 or an amino acid sequence that has a sequence identity of at least 60%, or at least 80% with SEQ ID No 9.

A pharmaceutical composition comprising the agent for use or the peptide for use as defined herein and in particular above and a pharmaceutically acceptable excipient is also provided.

The strategy herein described may provide effective therapy of both PD and MSA due to the influence of TPPP on the homo-association of SYN, and the hetero association of SYN (destruction of the SNY-TPPP assembly).

Another aspect of the invention is based on the finding that although SC-D is capable of inhibiting the homo-association of SYN (SYN-SYN aggregates), its effect on SYN-SYN aggregates in the presence of TPPP (Figure 7) is enhanced. This suggests the formation of SYN-TPPP aggregates in the presence of TPPP and SC-D. Furthermore although SC-D is capable of reducing (disaggregating) established SYN-SYN aggregates, its disaggregating effect on SYN-TPPP aggregates was found to be non-significant. These findings strongly suggest that a combination treatment of synucleinopathies or Parkinsonism, in particular PD or MSA with an agent targeting the homo-association of SYN and an agent targeting the hetero-association of SYN (SYN-TPPP aggregates) may be much more beneficial and effective than previously thought. Targeting the homo-association of SYN alone may prove to be insufficient, because the disaggregated SYN protein molecules hetero-associate with TPPP.

The above findings offer a solution for the unknown situation occurring in the pathological brain inclusions, namely, that the ratio/amount of the homo- and hetero-complexes of SYN may differ from patient to patient, depending on internal and external factors. Inhibiting the SYN-TPPP association and/or targeting both the SYN-SYN and SYN-TPPP association with a combination treatment eliminate the need to determine the ratio of the homo- and hetero-complexes of SYN in a patient without compromising the effectiveness of the treatment.

In this aspect a combination of two agents for use in the treatment of a synucleinopathy, preferably in the treatment of Parkinsonism, preferably in the treatment of PD or DLB or MSA by inhibiting the formation of or disintegrating both the SYN-SYN and the SYN-TPPP associations is provided. One of the agents is an agent capable of inhibiting the formation of or disrupting the association of SYN-SYN (SYN-SYN aggregates) and the other agent is capable of inhibiting the formation of or disrupting the association of SYN-TPPP. In another embodiment one of the agents is an agent capable of inhibiting the formation of or disrupting the associa-tion of SYN-SYN (SYN-SYN aggregates), preferably SC-D and the other agent is TPPP or a fragment of TPPP capable of binding to SYN.

A method is also provided for the treatment of a synucleinopathy, preferably in the treatment of Parkinsonism, preferably in the treatment of PD or DLB or MSA by inhibiting the formation of or disintegrating both the SYN-SYN and the SYN-TPPP associations, the method comprising administering to a patience in need thereof an an agent capable of inhibiting the formation of or disrupting the association of SYN-SYN (SYN-SYN aggregates) and an agent capable of inhibiting the formation of or disrupting the association of SYN-TPPP

The combination of two or at least two agents preferably has a synergistic effect (relative to the effect of either of the individual agent of the combination).

Highly preferably the agent capable of inhibiting the formation of or disrupting the association of SYN-SYN is SynuClean-D (SC-D, 1, 2-dihydro-5-nitro-6-(3-nitrophenyl)-2-oxo-4- (trifluoromethyl)-3-pyridinecarbonitrile, CAS No 685121-45-3) or a pharmeceutically acceptable salt, isomer or solvate thereof. Preferably the agent capable of inhibiting the formation of or disrupting the association of SYN-TPPP is an agent capable of inhibiting the formation of or disrupting the association of SYN-TPPP at the binding interface of SYN and TPPP.

Preferably the agent capable of inhibiting the formation of or disrupting the association of SYN-TPPP is a peptide comprising the amino acid sequence of SEQ ID NO 8.

Preferably the agent capable of inhibiting the formation of or disrupting the association of SYN-TPPP is a peptide comprising the amino acid sequence of SEQ ID NO 1 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 1.

Preferably the agent capable of inhibiting the formation of or disrupting the association of SYN-TPPP is a peptide comprising the amino acid sequence of SEQ ID NO 1 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 1 and the amino acid sequence of SEQ ID NO 8.

Highly preferably the agent capable of inhibiting the formation of or disrupting the association of SYN-TPPP is a peptide comprising the amino acid sequence of SEQ ID NO 2 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 2.

Preferably the agent capable of inhibiting the formation of or disrupting the association of SYN-TPPP is a peptide comprising the amino acid sequence of SEQ ID NO 3 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 3.

Preferably the agent capable of inhibiting the formation of or disrupting the association of SYN-TPPP is a peptide comprising the amino acid sequence of SEQ ID NO 4 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 4.

Preferably the agent capable of inhibiting the formation of or disrupting the association of SYN-TPPP is a peptide comprising the amino acid sequence of SEQ ID NO 5 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 5.

Preferably the peptide is up to 750 amino acid(aa) long, more preferably up to 500 aa long, more preferably up to 400 aa or up to 300 aa long.

A pharmaceutical composition comprising the agent for use or the peptide for use or the combination of two agents for use as defined herein and in particular above and a pharmaceutically acceptable excipient is also provided.

### EXAMPLES

### SYN

SYN expression and purification were performed as previously described [Oláh et al. Interactions of pathological hallmark proteins: tubulin polymerization promoting protein/p25, beta-amyloid, and alpha-synuclein. J Biol Chem. 2011 Sep 30;286(39):34088-100.].

The S129D mutant SYN was produced by recombinant technique. The pET21c SYN S129D mutant construct was amplified by polymerase chain reaction (PCR), using the forward primer 5'-GAAGGAGATATACAT-ATGGATGTATTCATGAAAGGAC-3' and the reverse primer 5'-ATATCTCGAGGGCTTCAGGTTCCGTAG-TCTTGATACCCTTCCTCATCAGGCATTTCATAAGCCTCAT TGTC-3', and the pT7-7 SYN wild type (addgene 36046) as a template. The PCR product was digested by NdeI and XhoI restriction enzymes, then the insert was ligated into pET21c (Novagen) vector. After sequencing, it was transformed into E. coli BL21 (DE3) cells. Other mutants were purchased from addgene (Addgene.org), pT7-7 SYN S129A (addgene 36048), pT7-7 SYN S87E (addgene 36059), pT7-7 SYN A30P (addgene 105726), pT7-7 SYN A53T (addgene 105727), pT7-7 SYN E46K (addgene 105728), and pT7-7 SYN G51D (addgene 105747). The purified plasmid DNA was transformed into E. coli BL21 (DE3) cells.

Human recombinant SYN forms were prepared as described in (Szunyogh et al., 2015). The SYN forms were expressed in ampicillin-resistant E. coli BL21 DE3 cells. Following transformation, BL21-competent cells were grown in LB in the presence of ampicillin (100 µg/ml). Cells were induced with 1 mM isopropyl β-D-1-thiogalactopyranoside, cultured at 37 °C for 2.5 h and harvested by centrifugation (20 min, 4 °C, 2000 g). Briefly, the cell pellet was resuspended in buffer A (20 mM Tris-HCl buffer pH 8.0 containing 1 mM ethylenediaminetetraacetic acid and 50 mM NaCl) with 5 mM 2-mercaptoethanol, 10 µM 4-(2-aminoethyl)benzenesulfonyl fluoride hydrochloride, 1 mM benzamidine, 1 µg/ml pepstatin and 1 µg/ml leupeptin; and lysed by sonication. After centrifugation (25min, 4 °C, 39,000 g), the cell suspension was kept at 90 °C for 10 min, and centrifuged again (25min, 4 °C, 39,000 g). The supernatant was loaded to a DE52 column equilibrated with buffer A. After extensive washing, SYN was eluted with 20 mMTris-HCl buffer pH 8.0 containing 1 mM ethylenediaminetetraacetic acid and 150 mM NaCl. The proteins were concentrated with a YM 3 Diaflo membrane (Amicon, Danvers, MA), dialyzed overnight in 50 mM NH4-acetate, lyophilized and stored at - 80 °C. The purity of proteins was analyzed by sodium dodecyl sulfate polyacrylamide gel electrophoresis (SDS-PAGE). Protein concentration was calculated from the absorbance at 280 nm using an extinction coefficient of 5960 M-1 *cm-1 for all SYN forms, since the mutations do not affect Tyr, Trp or Phe. Extinction coefficients were calculated using ProtParam (http://web.expasy.org/cgi-bin/protparam/protparam).

### TPPP

Human recombinant full length and double truncated TPPP/p25 forms possessing a His-tag were expressed in Escherichia coli BL21 (DE3) cells and isolated on HIS-Select^{™} Cartridge (Sigma-Aldrich) as described previously. Protein concentrations were determined from the absorbance at 280 nm using an extinction coefficient of 10,095 M-1 *cm-1 and 5625 M-1 *cm-1 for TPPP/p25 and double truncated TPPP/p25, respectively.

### Enzyme-linked immunosorbent assay (ELISA)

The plate was coated with 5 µg/ml (50 µl/well) TPPP/p25 or SYN forms in phosphate buffered saline (PBS: 10 mM Na2HPO4, 140 mM NaCl, pH 7.3). Briefly, after blocking the wells with bovine serum albumin (BSA), the immobilized proteins were incubated with serial dilutions of SYN forms or TPPP followed by the addition of either SYN (Sigma S3062, produced in rabbit) or TPPP antibody (polyclonal antibody Kovacs et al. 2004, produced in rat) and the corresponding peroxidase conjugated secondary IgG (anti-rabbit IgG-POD TermoFisher Scientific 32260 or anti-rat IgG-POD Sigma A9037). The bound antibodies were detected using o-phenylenediamine as substrate. The reaction was stopped after 10 min with 1 M H2SO4, and the absorbance was read at 490 nm with an EnSpire Multimode Reader (Perkin Elmer). The binding constants (Kd) were evaluated from the saturation curves by non-linear curve fitting assuming single binding site hyperbola model using the Origin 8.0 software. In the case of the competitive ELISA, 125 or 250 nM TPPP preincubated for 30 min without or with 1 µM SYN forms at constant concentration was added to the immobilized SYN. The bound TPPP was quantified by anti-TPPP antibody as described above. The results were normalized to that obtained by TPPP without SYN.

### Thioflavin assay

SYN expression and purification were performed as previously described [Oláh et al. Interactions of pathological hallmark proteins: tubulin polymerization promoting protein/p25, beta-amyloid, and alpha-synuclein. J Biol Chem. 2011 Sep 30;286(39):34088-100.]. The aggregation of SYN was carried out in a 2 ml Eppendorf tube. Each tube contained 100 µM SYN in PBS buffer, with or without TPPP (25-150 µM) and 1 borosilicate, 2 mm diameter solid-glass bead (Z273627-1EA, Merck) in a final volume of 250 µl. The tubes were kept in a shaker (200 rpm, 37C) for 2 days. At different time points, 5 µl aliquots were taken, and 100 µl 30 µM thioflavin-T solution was added to each aliquot in a black plate. The fluorescence was measured by exciting through a 450-15 nm filter and collecting the emission signal with a 515-30 filter, using matrix scan (5x5, 6 mm scan width) by a Clariostar plate reader (BMG).

### Enzyme-linked immunosorbent assay (ELISA)

The plate was coated with 5 µg/ml (50 µl/well) TPPP/p25 or SYN forms in phosphate buffered saline (PBS: 10 mM Na2HPO4, 140 mM NaCl, pH 7.3). Briefly, after blocking the wells with bovine serum albumin (BSA), the immobilized proteins were incubated with serial dilutions of SYN forms or TPPP followed by the addition of either SYN (Sigma S3062, produced in rabbit) or TPPP antibody (polyclonal antibody Kovacs et al. 2004, produced in rat) and the corresponding peroxidase conjugated secondary IgG (anti-rabbit IgG-POD TermoFisher Scientific 32260 or anti-rat IgG-POD Sigma A9037). The bound antibodies were detected using o-phenylenediamine as substrate. The reaction was stopped after 10 min with 1 M H2SO4, and the absorbance was read at 490 nm with an EnSpire Multimode Reader (Perkin Elmer). The binding constants (Kd) were evaluated from the saturation curves by non-linear curve fitting assuming single binding site hyperbola model using the Origin 8.0 software.

### Competitive ELISA

125 or 250 nM TPPP preincubated for 30 min without or with 1 µM SYN forms at constant concentration was added to the immobilized SYN. The bound TPPP was quantified by anti-TPPP antibody as described above. The results were normalized to that obtained by TPPP without SYN.

### cELISA

HeLa cells plated in 96-wells and propagated overnight. Next day, different amounts of SYN or SYN-TPPP mix were added to the medium of wells and incubated for 4 hours. In the case of peptides activity, 1 µM SYN and 0.5 µM TPPP mixed in PBS and added to the cells. The different peptide samples were added to the cells before the mix in 50 µM concentration. After 4 hours, the cells were fixed by cold methanol and the wells were blocked by 5% FCS-0.1% TXT-100 in PBS. The level of SYN in cells detected by a commercial antibody SYN-201 in 1:1500 dilution, 50 µl per well. The secondary antibody was anti-mouse IgG conjugated by HRPO in 1:2500 dilution, 50 µl per well. HRPO activity was measured in the wells by H₂O₂-OPD substrate, 100 µl per well, stopped by 25 µl per well 1 N H₂SO₄ after 30 minutes and measured by 492 nm absorbance by a Clariostar plate reader (BMG). Methodology for cell culture, transfection, manipulation and immunofluorescence confocal microscopy with or without Bimolecula Fluorescence Complementation (BiFC) are described as follows: Szénási T, Oláh J, Szabó A, Szunyogh S, Láng A, Perczel A, Lehotzky A, UverskyVN, Ovádi J. Biochim Biophys Acta Mol Basis Dis (2017) 63:310-323; Lehotzky A, Oláh J, Fekete JT, Szénási T et al Frontiers in Molecular Biosciences (2021)8:666026; Szunyogh S, Oláh J, Szénási T, Szabó A, Ovádi J Biochim Biophys Acta. (2015) 1852:2653-61

### Statistical Analysis

The values are presented as the mean ± SD (standard deviation) of at least 3 independentc experiments, or a box diagram is shown. Statistical comparisons were performed with one-way ANOVA followed by Tukey's test for multiple comparisons using Origin 2018 64-bit software. Values were considered to be significant if the calculated p value was <0.05 (* p < 0.05, ** p < 0.01, ***p < 0.001 and **** p < 0.0001

## Claims

1. An agent for use in inhibiting the formation of or disintegrating the alpha-synuclein - alpha-synuclein (SYN-SYN) complex, wherein the agent inhibits or disrupts the association of tubulin polymerization promoting protein (TPPP) and SYN at the interface of the SYN-TPPP complex.

2. An agent for use according to claim 1, wherein the agent binds to the amino acid sequence of SGVTK (SEQ ID NO 9) in TPPP, thereby inhibiting the formation of or disintegrating the SYN-TPPP complex.

3. An agent for use according to claim 1, wherein the agent binds to the amino acid sequence of SEQ ID NO 1 in TPPP, thereby inhibiting the formation of or disintegrating the SYN-TPPP complex.

4. The agent for use according to claim 1, wherein the agent comprises the amino acid sequence of SEQ ID NO 8.

5. The agent for use according to claim 1, wherein the agent comprises the amino acid sequence of SEQ ID NO 1 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 1.

6. The agent for use according to claim 1, wherein the peptide comprises the amino acid sequence of SEQ ID NO 2 or an amino acid sequence having at least 75% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with SEQ ID NO 2.

7. The agent for use according to any one of the preceding claims, wherein the agent is for use in the treatment or prevention of a synucleinopathy.

8. The agent for use according to claim 7, wherein the synucleinopathy is Parkinsonism.

9. The agent for use according to claim 8, wherein the synucleinopathy is Parkinson's disease (PD) or Multiple System Atrophy (MSA).

10. A pharmaceutical composition comprising the agent for use according to any one of the preceding claims and a pharmaceutically acceptable carrier or excipient.

11. A combination of at least two agents for use in the treatment of a synucleinopathy, wherein one of the agents inhibits the formation of or disrupts the association of SYN-TPPP and the other agent inhibits the formation of or disrupts the association of SYN-SYN.

12. The combination for use according to claim 11, wherein the agent inhibiting the formation of or disrupting the association of SYN-TPPP inhibits or disrupts the association of SYN-TPPP at the interface of the SYN-TPPP complex.

13. The combination for use according to claim 11 or 12, wherein the agent inhibiting the formation of or disrupting the association of SYN-SYN is 2-hydroxy-5-nitro-6-(3-nitrophenyl)-4-(trifluoromethyl)nicotinonitrile (SC-D).

14. The combination for use according to any one of claims 11-13, wherein the agent inhibiting the formation of or disrupting the association of SYN-TPPP binds to or disrupts the amino acid sequence of SGVTK (SEQ ID NO 9) in TPPP.

15. The combination for use according to any one of claims 11-14, wherein the agent inhibiting the formation of or disrupting the association of SYN-TPPP comprises an amino acid sequence selected from the group consisting of SEQ ID NOs 1-5 and 8-10 and amino acid sequences having at least 70% sequence identity, preferably at least 80% sequence identity, preferably at least 85% sequence identity, preferably at least 90% sequence identity, preferably at least 95% sequence identity, preferably at least 96% sequence identity, preferably at least 97% sequence identity, preferably at least 98% sequence identity with any one of SEQ ID NOs 1-5 and 8-10.
